# EUROPÄISCHE PATENTSCHRIFT

(11) **EP 0 555 270 B1**
(45) Veröffentlichungstag und Bekanntmachung des Hinweises auf die Patenterteilung: **03.05.1995**
(21) Anmeldenummer: 91918495.2
(22) Anmeldetag: 24.10.1991
(51) Int. Cl.: C07H 1/08

(54) **VERFAHREN ZUR ISOLIERUNG VON NUKLEINSÄUREN AUS ZELLSUSPENSIONEN**
PROCESS FOR INSULATING NUCLEIC ACIDS FROM CELL SUSPENSIONS
PROCEDE DE SEPARATION D'ACIDES NUCLEIQUES CONTENUS DANS DES SUSPENSIONS DE CELLULES

(30) Priorität: 26.10.1990 DE 4034036
(43) Veröffentlichungstag der Anmeldung: 18.08.1993
(73) Patentinhaber: QIAGEN GmbH, 40724 Hilden (DE)
(72) Erfinder: HENCO, Karsten, D-4006 Erkrath 2 (DE); COLPAN, Metin, D-4300 Essen-Kettwig (DE); FEUSER, Petra, D-5000 Köln 41 (DE)
(74) Vertreter: Meyers, Hans-Wilhelm, Dr.Dipl.-Chem.
(86) Internationale Anmeldenummer: EP9102017
(87) Internationale Veröffentlichungsnummer: WO9207863

(56) Entgegenhaltungen:
- EP-A- 0 268 946

## Beschreibung

Gegenstand der Erfindung ist ein Verfahren zur Isolierung von Nukleinsäuren durch Lysis intakter Zellen und Entfernung der aus den lysierten Zellen ausgetretenen Nukleinsäuren sowie eine Vorrichtung zur Durchführung dieses Verfahrens.

Die Reinigung von Nukleinsäuren spielt eine zentrale Rolle in der modernen Molekularbiologie. Nukleinsäuren dienen als Ausgangsmaterial für Genklonierungen und genetische Analysen in der labordiagnostischen Forschung und im routinemäßigen Einsatz. Zum Beispiel wird auf Nukleinsäurebasis die Analyse genetischer Erkrankungen von Blutzellen, Virus- und Bakterienanalytik aus Blut, Urin, Stuhl oder Sekreten durchgeführt. Der Analyse aus Vollblut kommt dabei besondere klinische Bedeutung zu.

Üblicherweise werden Nukleinsäuren aus Zellen gewonnen. Dabei werden Zellen beispielsweise unter stark denaturierenden und gegebenenfalls reduzierenden Bedingungen aufgeschlossen (Maniatis, T., Fritsch, E.F. & Sambrook, S., 1982, Molecular Cloning: A Laboratory Manual, Cold Spring Harbor University Press, Cold Spring Harbor). Weit verbreitet ist der Aufschluß der Zellen mit Detergenzien als denaturierende Reagenzien und die Verwendung von bestimmten Enzymen zum Abbau der Proteinstukturen und nukleinsäurespaltenden Enzymen. So werden beispielsweise Natriumdodecylsulfat (SDS) und Ethylendiamintetraessigsäure (EDTA) als denaturierende Agenzien sowie Enzyme wie Proteinase K verwendet. Das Ergebnis dieses Aufschlußverfahrens ist meistens eine hochviskose gallertartige Struktur, aus der die Nukleinsäuren mittels Phenolextraktion isoliert werden. Die Nukleinsäuren bleiben dabei in großer Länge erhalten und werden nach Dialyse und Präzipitation aus der wässrigen Phase entfernt. Dieses Aufschlußverfahren ist gegenüber Nicht-Nukleinsäure-Strukturen so aggressiv, daß dem Verfahren auch Gewebestücke unterworfen werden können. Wegen der arbeitsintensiven Technik mit mehrfachem Wechsel der Reaktionsgefäße ist diese Methode jedoch für große Probenaufkommen und Rountinepräparationen ungünstig. Dieses Verfahren ist zwar automatisierbar, jedoch bewältigt eine handelsübliche Apparatur gegenwärtig etwa 8 Proben gleichzeitig in vier Stunden (Applied Biosystems A 371). Das Verfahren ist somit teuer und eignet sich nicht für die Durchschleusung großer Probenserien. Weiterhin ist nachteilig, daß Folgereaktionen, wie enzymatische Amplifikation, infolge der großen Längen der isolierten Nukleinsäuren gestört werden. Darüber hinaus sind die anfallenden Lösungen hochviskos und schwer handhabbar. Insbesondere DNA sehr großer Länge ist eher störend, da die mit dem Verfahren gemäß Stand der Technik gewonnenen Nukleinsäuren gesondert zerkleinert werden müssen, um weiterverarbeitet werden zu können.

Der Aufschluß von Zellen im alkalischen Milieu in Gegenwart von Detergenzien, ist technisch zwar einfach, liefert jedoch auch Nukleinsäuren mit großer Länge.

An die Rohpräparation der Nukleinsäuren schließen sich Folgereaktionen an. Diese Folgereaktionen erfordern eine bestimmte Qualität der Nukleinsäuren. So muß die Nukleinsäure weitestgehend unversehrt, die Ausbeute der Präparation muß hoch und reproduzierbar sein. Der Präparationsweg muß einfach und wirtschaftlich sein und die Möglichkeit zur Automation bieten. Die Präparation der Nukleinsäure muß ohne die Gefahr der Kreuzkontamination anderer Proben möglich sein , insbesondere wenn enzymatische Amplifikationsreaktionen, wie Polymerase Chain Reaction (PCR) (Saiki, R., Gelfand, D.H., Stoffel, S., Scharf, S.J., Higuchi, R., Horn, G.T., Mullis, K.B. & Erlich, H. A. (1988), Science 239, 487 - 491) und Ligase Chain Reaction (LCR) (EP-A-88 311 741.8), Anwendung finden. Daher ist es wünschenswert, die Nukleinsäuren in nicht zu großer Kettenlänge zu erhalten, die Zellen möglichst quantitativ aufzuschließen und im übrigen die oben genannten Nachteile der im Stand der Technik bekannten Aufschlußverfahren zu vermeiden.

Die EP 0 268 946 betrifft ein Verfahren zur Trennung langkettiger Nukleinsäuren. Dabei werden langkettige Nukleinsäuren nach einem erfolgten Zellaufschluß unter Bedingungen niedriger Ionenstärke auf einem Anionenaustauschergel adsorbiert, gegebenenfalls gewaschen und dann mit einem Lösungsmittelsystem hoher Ionenstärke eluiert. Es wird eine Vorrichtung beschrieben, bei der in einer Kartusche angeordnete Ionenaustauscher durch eine Einlaßöffnung mit der nukleinsäurehaltigen Probe beschickt wird und nach den Reinigungs- und Trennoperationen durch die Auslaßöffnung 4 wieder verläßt.

Das der Erfindung zugrundeliegende technische Probleme besteht in der Bereitstellung eines verbesserten Verfahrens zur Isolierung von Nukleinsäuren aus intakten Zellen, insbesondere soll die dabei anfallende Nukleinsäure sich durch nicht zu große Kettenlängen auszeichnen, in wenigen Schritten isolierbar sein und direkt den erforderlichen Folgereaktionen unterworfen werden können.

Dieses technische Problem wird gelöst durch ein Verfahren zur Isolierung von Nukleinsäuren durch Lysis intakter Zellen und Entfernung der aus den lysierten Zellen ausgetretenen Nukleinsäuren, dadurch gekennzeichnet, daß
a) die Zellen in einer porösen Matrix immobilisiert werden, wobei die Größe der Hohlräume der Matrix in der Größenordnung des zu lysierenden Zelltyps liegt,
b) die Zellen lysiert werden,
c) die Nukleinsäuren auf der Matrixoberfläche fixiert werden und danach
d) eluiert werden.

Die Matrix besteht vorzugsweise aus porösen, anorganischen oder organischen Polymeren, wobei die Oberflächeneigenschaften des die Matrix bildenden Materials dafür sorgen, daß die Zellen reversibel oder irreversibel an der Oberfläche immobilisiert werden. Vorzugsweise beträgt die Größe der Hohlräume des die Matrix bildenden Materials 1 bis 50 »m. Besonders bevorzugt sind Hohlraumgrößen von 5 bis 15 »m. Erreicht werden kann dies in einer bevorzugten Ausführungsform des erfindungsgemäßen Verfahrens, wenn die Partikelgröße des die Matrix bildenden Materials 15 bis 25 »m beträgt.

Die in der Matrix immobilisierten Zellen werden vorzugsweise durch physikalische oder chemische Einwirkung lysiert. Dabei kann die Lyse entweder mechanisch wie mit Ultraschall oder durch Scherkräfte, osmotischem Schock oder chemisch mittels Detergenzien oder alkalischem Aufschluß erreicht werden.

In einer besonders bevorzugten Ausführungsform weist die Oberfläche des die Matrix bildenden Materials Ionenaustauschereigenschaften auf. Insbesondere bei Verwendung von Anionenaustauschern kann dann die aus der lysierten Zelle austretende Nukleinsäure reversibel an die Oberfläche des die Matrix bildenden Materials gebunden werden, um nach verschiedenen Waschoperationen dann durch Einstellen hoher Ionenstärken eluiert zu werden.

Die erfindungsgemäße Verfahrensweise führt zur Präparation von Nukleinsäuren mit hoher Reinheit und erlaubt es, eine qualitativ und quantitativ reproduzierbare Analytik durchzuführen, insbesondere in Kombination mit enzymatischen Verfahren zur Amplifikation von Nukleinsäuren. Es hat sich gezeigt, daß milde Aufschlußmethoden mit Detergenzien oder physikalische Aufschlußmethoden, wie Erhitzen einer Probe, die Folgeanwendungen erleichtert. Man erhält bei Anwendung des erfindungsgemäßen Verfahrens zum Beispiel kürzere zelluläre DNA (< 200 kb) beziehungsweise Gesamtnukleinsäuren aus der Zelle. Die Ursachen für eine limitierte Spaltung der Nukleinsäuren sind nicht vollständig bekannt. Die milden Aufschlußmethoden scheinen jedoch zu einer partiellen Degradation der Nukleinsäure zu führen.

Für die reproduzierbare Arbeitsweise ist es vorteilhaft, eine Matrix mit Ionenaustauschereigenschaften zu verwenden, so daß die freigesetzten Nukleinsäuren an dieser Oberfläche haften können und dem Zugriff abbauender Enzyme entzogen sind. Es werden dann Nukleinsäuren mit fast idealer Länge für die Folgereaktionen, wie PCR, gewonnen.

Sollen zum Beispiel weiße Blutzellen von Säugern aufgeschlossen werden, müssen zunächst die DNA-freien Erythrozyten entfernt werden. Verschiedene Verfahren dazu sind bekannt, wie zum Beispiel Gradientenzentrifugation oder Extraktion der Zellen mittels oberflächengekoppelter Antikörper (anti-HLA-Magnetobeads). Als besonders vorteilhaft hat sich erwiesen, die Erythrozyten mittels Dextran zu agglomerieren und ohne Zentrifugation abzutrennen. Der Überstand enthält die anderen Blutzellen und weniger als 1/100 der anfänglichen Erythrozytenkonzentration. Die weißen Blutzellen werden dann nach dem erfindungsgemäßen Verfahren aus der Suspension in die Poren der Matrix überführt. Dies geschieht durch Druckdifferenz oder Zentrifugationskräfte. Es hat sich herausgestellt, daß es nicht genügt, Zellen beispielsweise durch einfache Zentrifugation, durch Ausbildung eines Pellets oder durch eine Filtration über engmaschige Membranen zu konzentrieren, da dann der erfindungsgemäße Zellaufschluß nicht mehr zuverlässig erfolgt. Dadurch entstehende, relativ dichte Zellverbände mit direktem Zell/Zell-Kontakt können nur unzureichend mit den milden nicht-ionischen Reagenzien aufgeschlossen werden. Es werden dabei wahrscheinlich nur diejenigen Zellen lysiert, die sich an der Oberfläche eines solchen Zellkonzentrats befinden.

Durch das erfindungsgemäße Verfahren wird sichergestellt, daß eine solche Pelletierung nicht einsetzt. Hierbei werden die Zellen durch eine Art Tiefenfiltration in der Matrix konzentriert, d. h. in einem kleinen Volumenelement eingefangen. Das bedeutet, daß Zellen praktisch isoliert in der Matrix angeordnet sind, jedoch nicht übereinander liegen. Erfindungsgemäß erreicht wird dies durch die Verwendung einer porösen Matrix zum Beispiel einem Partikelbett, dessen Zwischenräume in der Größenordnung der Zellgröße liegen. Die Zellen dringen dann bis zu einer bestimmten Tiefe in diese Matrix ein.

Bei einer bevorzugten Ausführungsform des erfindungsgemäßen Verfahrens kommen Silicalgelpartikel zum Einsatz mit einer Partikelgröße von vorzugsweise 15 bis 25 »m, so daß Zwischenkornabstände von 1 bis 50 »m, vorzugsweise 5 bis 15 »m, entstehen. Die durchschnittliche Größe von Erythrozyten beträgt 2 »m, von Lymphozyten ungefähr 8 »m und von Monozyten etwa 15 »m.

In einer weiteren bevorzugten Ausführungsform des erfindungsgemäßen Verfahrens werden zur Immobilisierung Partikel verwendet, die in einem inerten Netzwerk aus Membranen, vorzugsweise Teflon, eingebettet sind, wobei die Zwischenräume wiederum in etwa der Dimension der zu lysierenden Zellen entsprechen. Dadurch wird gewährleistet, daß sich die Zellen vorzugsweise innerhalb des Netzwerks festsetzen und sich nicht, zum Beispiel wie auf einem Sterilfilter, als dichte Packung in Form eines Filterkuchens absetzen.

Zwischen den Zellen verbleiben enge Kanäle, durch die Lösungen ausgetauscht werden können, wie zum Beispiel das Serum gegen die Zell-lysierende Lösung. Da alle Zellen für Reagenzien gut zugänglich sind, können die milden Lysebedingungen Verwendung finden, ohne daß Ausbeuteverluste zu befürchten sind.

In einer besonders bevorzugten Ausführungsform weisen die Partikel Ionenaustauschereigenschaften auf, wie sie beispielsweise in der DE-PS 32 11 305 beschrieben werden. Für die Ausführungsform auf Membranbasis hat sich ein Ionenaustauscher auf Silicagelbasis mit Partikelgrößen zwischen 15 und 25 »m und Porengrößen von 1500 Å als besonders geeignet erwiesen. Der direkte räumliche Kontakt zu allen lysierenden Zellen gewährleistet, daß die DNA nach der Lyse und partiellen Degradation direkt an der Oberfläche der Festphase fixiert wird und so vor weiterführenden Angriffen der Nukleasen geschützt ist. In diesem Zustand können die kontaminierenden Bestandteile leicht ausgewaschen werden, gefolgt von der Elution der sauberen Nukleinsäure in einem kleinen Volumen. Man erhält so reproduzierbare Kettenlängen von durchschnittlich 5 bis 20 kb. Weniger als 10% sind kürzer als 5 kb unter den Aufschlußbedingungen, wie im Beispiel beschrieben. Dies stellt eine optimale Längenverteilung für eine anschließende enzymatische Nukleinsäureamplifikation dar. Es wurde ebenfalls überraschend festgestellt, daß die gewünschte Größenverteilung bei der Leukozytenpräparation mit Dextran reproduzierbar erreicht wird, während eine Präparation über Ficoll im Gradienten gewöhnlich zu kürzeren Fragmenten (500 bis 1000 bp) führt.

Es hat sich herausgestellt, daß das erfindungsgemäße Verfahren in besonders günstiger Weise mittels einer Vorrichtung, die im folgenden beschrieben werden soll, durchgeführt werden kann. Die Vorrichtung besteht aus einem Hohlkörper (4), in dem die die Zellen aufnehmende Matrix (1) zwischen 2 porösen Einrichtungen (2, 3) angeordnet ist. Die Porengröße der Einrichtung 2, 3, vorzugsweise Polyethylen- oder Glasfritten, muß dabei größer sein als die Hohlraumgröße des die Matrix 1 bildenden Materials. Die Einrichtungen 2, 3 haben eine Porengröße von 50 bis 100 »m, wobei die Hohlraumgröße des die Matrix 1 bildenden Materials etwa 1 bis 50 »m beträgt. Die Matrix 1 ist eine netzartige Membran mit einer Vielzahl von 1 bis 50 »m großen freien Poren, die zusätzlich Ionenaustauscherpartikel aufweist. In den freien Poren werden dann die Zellen immobilisiert, wobei auf den in dieser Matrix befindlichen Ionenaustauscherpartikeln die freigesetzten Nukleinsäuren adsorbiert werden.

Eine ebenfalls bevorzugte Ausführungsform stellt eine Vorrichtung dar, bei der das die Matrix 1 bildende Material ein partikelförmiges organisches oder anorganisches Polymeres ist. Vorzugsweise ist das die Matrix 1 bildende Material ein Kieselgel mit Ionenaustauschereigenschaften und einer Partikelgröße von 15 bis 25 »m.

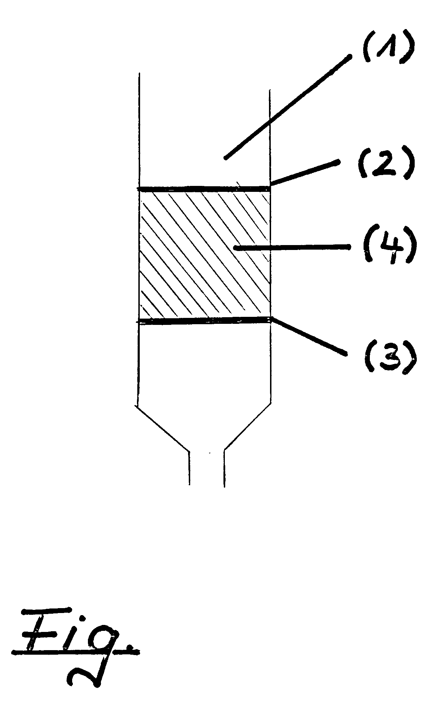

Die Abbildung zeigt ein bevorzugtes Ausführungsbeispiel der Vorrichtung. Der Hohlkörper 4 kann zum Beispiel ein handelsübliches Röhrchen (MoBiCol™, MoBiTec™, Göttingen oder Millipore^{R} UFC3 OHW25) sein. Zwischen zwei eng eingepreßten Einrichtungen 2, 3, zum Beispiel Polyethylenfritten mit einer Porengröße von 50 bis 100 »m, befindet sich eine Membran mit 5 bis 10 »m großen freien Poren, welche ebenfalls Kieselgel (1500 Å Porengröße, 15 »m Partikelgröße) mit Anionenaustauschereigenschaften enthält. Die Membran weist eine Dicke von ca. 1 mm auf. Die Kapazität des Ionenaustauschermaterials ist etwa 15 »g DNA. Durch Übereinanderlegen entsprechender Membranen läßt sich selbstverständlich die Kapazität für DNA erhöhen. Bei geringer mechanischer Belastung ist auch ein randständiges Verschweißen oder Verkleben der Membran denkbar, wodurch die stabilisierende Wirkung der Einrichtungen 2, 3 entfallen kann, so daß die Membran den Hohlkörper 4 ohne die Einrichtungen verschließt.

Es ist ebenfalls möglich, mit dem beschriebenen Kieselgel kleine Säulen zu füllen, das zwischen 2 Polyethylen-Fritten mit einer Porengröße von 35 »m angeordnet ist. Vorzugsweise wird die obere Einrichtung 2 mit größeren Poren (70 »m) gewählt. Die MoBiCol-Säulen werden mit ca. 70 mg Ionenaustauschergel entsprechend 3 mm Füllhöhe beschickt. Bezüglich Plasmid-DNA ergibt sich eine Kapazität von 150 »g DNA.

### Beispiel

### Zelluläre DNA-Präparation aus Blut:

6 ml Citrat-Blut werden in einem 12 ml PPN-Röhrchen mit 0,12 g Dextran (MW 250.000) homogen versetzt und für 60 min. bei Raumtemperatur inkubiert. Dextran und Erythrozyten bilden Aggregate, die innerhalb 45 min. sedimentieren. Der helle Überstand enthält die Leukozyten annähernd in der natürlichen Zusammensetzung ((1 - 5) x 10⁶ Leukozyten/ml, Erythroz./Leukoz. ca. 2/1). 0,5 ml dieses Überstandes werden auf die Vorrichtung aufgetragen und durch Zentrifugation oder Druck durch den Filter geleitet. Die Zellen werden im Netzwerk zurückgehalten. Nach Durchtritt des Serums wird mit 1 x 0,5 ml Waschlösung (PBS 120 mM NaCl, 2,7 mM KCl, 10 mM KPi pH 7,4) (phosphate buffered saline) gewaschen.

Danach werden die Zellen mit 125 »l einer 1%igen Lösung eines nicht-ionischen Detergens (NP40; Tween 20) lysiert. Mit 0,5 ml 750 mM KCl, 50 mM MOPS pH 7,0, 15% Ethanol werden die Verunreinigungen ausgewaschen. Die DNA wird entweder mit einem Hochsalzpuffer (1,2 M KCl, 50 mM, MOPS, pH 7,0) oder einem Alkalipuffer (200 mM KOH) eluiert. Der Vorteil des Alkalipuffers besteht darin, daß nach Pufferzusatz zwecks Neutralisation beispielsweise direkt eine enzymatische Amplifikation erfolgen kann, ohne daß die Nukleinsäure zunächst gefällt werden muß. Es besteht jedoch die Gefahr der Schädigung der DNA. In vielen Fällen kann auch ein Aliquot der Hochsalzelution direkt eingesetzt werden, wenn die Nukleinsäurekonzentration die erforderliche Konzentration aufweist.

## Patentansprüche

1. Verfahren zur Isolierung von Nukleinsäuren durch Lysis intakter Zellen und Entfernung der aus den lysierten Zellen ausgetretenen Nukleinsäuren, dadurch gekennzeichnet, daß
a) die Zellen in einer porösen Matrix immobilisiert werden, wobei die Größe der Hohlräume der Matrix in der Größenordnung des zu lysierenden Zelltyps liegt,
b) die Zellen lysiert werden,
c) die Nukleinsäuren auf der Matrixoberfläche fixiert werden und danach
d) eluiert werden.

2. Verfahren nach Anspruch 1, dadurch gekennzeichnet, daß die Matrix ein anorganisches oder organisches Polymeres ist.

3. Verfahren nach Anspruch 1 und/oder 2, dadurch gekennzeichnet, daß die Matrix eine Hohlraumgröße von 1 bis 50 »m aufweist.

4. Verfahren nach Anspruch 3, dadurch gekennzeichnet, daß die Hohlräume 5 bis 15 »m groß sind.

5. Verfahren nach einem der Ansprüche 1 bis 4, dadurch gekennzeichnet, daß die Partikelgröße der Matrix 10 bis 50 »m, insbesondere 15 bis 25 »m beträgt.

6. Verfahren nach einem der Ansprüche 1 bis 5, dadurch gekennzeichnet, daß die Zellen durch physikalische oder chemische Einwirkungen lysiert werden.

7. Verfahren nach Anspruch 6, dadurch gekennzeichnet, daß die Lyse der Zellen durch Ultraschall, Scherkräfte, Detergenzien, Enzyme, osmotischer Schock oder Kombination davon erfolgt.

8. Verfahren nach einem der Ansprüche 1 bis 7, dadurch gekennzeichnet, daß die Oberfläche der Matrix Ionenaustauschereigenschaften aufweist.

9. Verfahren nach einem der Ansprüche 1 bis 8, dadurch gekennzeichnet, daß die Nukleinsäuren durch einen Puffer mit hoher Ionenstärke oder alkalisch eluiert werden.

10. Verfahren nach einem der Ansprüche 1 bis 9, wobei zwischen den Schritten a)/b) und/oder b)/c) und/oder c)/d) die Matrix gewaschen wird.

11. Verwendung einer Vorrichtung mit einer zellenaufnehmenden Matrix (1), die in einem Hohlkörper (4) zwischen 2 porösen Einrichtungen (2, 3) angeordnet ist, wobei die Porengröße der Einrichtungen (2, 3) größer ist als die Hohlraumgröße des die Matrix (1) bildenden Materials, zur Isolierung von Nukleinsäuren aus Zellen nach Aufschluß der Zellen gemäß einem der Ansprüche 1 bis 10.

12. Verwendung nach Anspruch 11, wobei die Porengröße der Einrichtungen (2, 3) 50 bis 100 »m und die Hohlraumgröße des die Matrix (1) bildenden Materials 1 bis 50 »m beträgt.

## Claims

1. A process for isolating nucleic acids by lysing intact cells and removing the nucleic acids emerging from the lysed cells, characterized in that
a) the cells are immobilized in a porous matrix, with the size of matrix voids being in the range of that of the type of cell to be lysed;
b) the cells are lysed;
c) the nucleic acids are fixed on the matrix surface; and subsequently
d) are eluted.

2. The process according to claim 1, characterized in that said matrix is an inorganic or organic polymer.

3. The process according to claim 1 and/or 2, characterized in that said matrix has a void size of from 1 to 50 »m.

4. The process according to claim 3, characterized in that the void size is from 5 to 15 »m.

5. The process according to any of claims 1 to 4, characterized in that the matrix particle size is from 10 to 50 »m, in particular from 15 to 25 »m.

6. The process according to any of claims 1 to 5, characterized in that the cells are lysed by physical or chemical action.

7. The process according to claim 6, characterized in that the cell lysing is effected by ultrasonic waves, shear forces, detergents, enzymes, osmotic shock, or combinations thereof.

8. The process according to any of claims 1 to 7, characterized in that the matrix surface has ion-exchange properties.

9. The process according to any of claims 1 to 8, characterized in that the nucleic acids are eluted by a buffer with high ionic strength or by alkaline elution.

10. The process according to any of claims 1 to 9, wherein said matrix is washed between steps a)/b) and/or b)/c) and/or c)/d).

11. Use of a device having a cell-accomodating matrix (1) arranged in a hollow body (4) between 2 porous units (2, 3), wherein the pore size of units (2, 3) is larger than the void size of the material forming matrix (1), for isolating nucleic acids from cells subsequent to cell digestion according to any of claims 1 to 10.

12. The use according to claim 11, wherein the pore size of units (2, 3) is from 50 to 100 »m and the void size of the material forming matrix (1) is from 1 to 50 »m.

## Revendications

1. Procédé pour isoler des acides nucléiques par lyse de cellules intactes et extractions des acides nucléiques hors des cellules lysées,
caractérisé en ce que :
a) on immobilise les cellules dans une matrice poreuse dont la taille des pores est de l'ordre de grandeurs de la taille des cellules à lyser
b) on lyse les cellules
c) on fixe les acides nucléiques sur la surface de la matrice et
d) on procède à l'élution desdits acides nucléiques.

2. Procédé selon la revendication 1, caractérisé en ce que la matrice est un polymère organique ou inorganique.

3. Procédé selon la revendication 1 et/ou 2, caractérisé en ce que la taille des pores de la matrice est de 1 à 50 micromètres.

4. Procédé selon la revendication 3, caractérisé en ce que la taille des pores est de 5 à 15 micromètres.

5. Procédé selon l'une des revendications 1 à 4, caractérisé en ce que la taille des particules de la matrice est de 10 à 50 micromètres, en particulier de 15 à 25 micromètres.

6. Procédé suivant l'une quelconque des revendications 1 à 5, caractérisé en ce que les cellules sont lysées par des agents physiques ou chimiques.

7. Procédé suivant la revendication 6, caractérisé en ce que la lyse des cellules est exécutée par des ultrasons, des forces de cisaillement, des détergents, des enzymes, des chocs osmotiques, ou par une combinaison de ces agents.

8. Procédé suivant l'une quelconque des revendications 1 à 7, caractérisé en ce que la surface de la matrice présente des propriétés d'échange d'ions.

9. Procédé suivant l'une quelconque des revendications 1 à 8, caractérisé en ce que l'élution desdits acides nucléiques est réalisée de façon alcaline ou au moyen d'un tampon à forte teneur ionique.

10. Procédé suivant l'une quelconque des revendications 1 à 9, caractérisé en ce que l'on lave la matrice entre les étapes a)/b) et/ou b)/c) et/ou c)/d).

11. Afin d'isoler des acides nucléiques hors de cellules après ouverture de celles-ci, selon l'une quelconque des revendications 1 à 10, utilisation d'un dispositif comprenant une matrice (1) réceptrice de cellules placée entre deux dispositifs poreux (2, 3) dans un corps creux (4), la taille des pores des dispositifs (2, 3) étant supérieure à la taille des pores du matériau formant la matrice (1).

12. Utilisation selon la revendication 11, dans laquelle la taille des pores desdits dispositifs (2, 3) est de 50 à 100 micromètres et en ce que la taille des pores du matériau formant la matrice (1) est de 1 à 50 micromètres.
